# FASCICULE DE BREVET EUROPEEN

(11) **EP 3 021 750 B1**
(45) Date de publication et mention de la délivrance du brevet: **09.09.2020**
(21) Numéro de dépôt: 14746979.5
(22) Date de dépôt: 17.07.2014
(51) Int. Cl.: A61B 5/157, B01L 3/02, G01N 35/10, A61B 5/15

(54) **DISPOSITIF ET PROCEDE D'ECHANTILLONNAGE ET DE DISTRIBUTION D'UN FLUIDE BIOLOGIQUE UTILISANT UN TUBE CAPILLAIRE, ET APPAREIL D'ANALYSE BIOLOGIQUE**
VORRICHTUNG UND VERFAHREN ZUR ENTNAHME UND ABGABE EINER BIOLOGISCHEN FLÜSSIGKEIT MITTELS EINES KAPILLARROHRES UND BIOANALYSE-VORRICHTUNG
DEVICE AND METHOD FOR SAMPLING AND DISPENSING A BIOLOGICAL FLUID USING A CAPILLARY TUBE, AND BIOLOGICAL ANALYSIS APPARATUS

(30) Priorité: 17.07.2013 FR 1357026
(43) Date de publication de la demande: 25.05.2016
(73) Titulaire: Horiba ABX SAS / Horiba Ltd, 34184 Montpellier cedex 4 (FR)
(72) Inventeur: CREMIEN, Didier, F-34990 Juvignac (FR); ISEBE, Damien, F-34090 Montpellier (FR)
(74) Mandataire: IPAZ
(86) Numéro de dépôt international: PCT/EP2014/065412
(87) Numéro de publication internationale: WO 2015/007853

(56) Documents cités:
- WO-A1-01/75416
- WO-A1-03/044488
- FR-A1- 2 774 765
- JP-A- 2003 159 235
- JP-A- 2008 275 353
- US-A- 4 133 304
- US-A- 4 653 511
- US-A- 5 638 828

## Description

### Domaine technique

La présente invention concerne un dispositif d'échantillonnage et de distribution d'échantillon biologique pour appareils d'analyse biologique.

Le domaine de l'invention est plus particulièrement mais de manière non limitative celui des systèmes d'analyse biologique, et notamment celui des systèmes d'analyse sanguine.

### Etat de la technique antérieure

De manière classique, les prélèvements d'échantillons sanguins pour des opérations d'analyse biologique sont effectués par ponction veineuse.

Le prélèvement veineux nécessite un protocole particulier réalisé par un préleveur ou un infirmier qualifié. Cette technique requiert le prélèvement d'une importante quantité de sang (environ 4 ml) auprès du patient. L'échantillon sanguin est collecté dans un tube classique.

Pour effectuer les analyses, le tube est ensuite introduit dans un analyseur sanguin. Une aiguille de prélèvement est introduite dans le tube, afin de transférer un certain volume du sang prélevé vers un système d'échantillonnage situé à l'intérieur de l'analyseur.

Le système d'échantillonnage est en général constitué d'une vanne d'échantillonnage ou d'une seringue. Il doit permettre d'isoler ou de prélever précisément une aliquote de sang d'un volume prédéfini qui est utilisé pour l'analyse.

D'une manière générale, le volume de sang effectivement utilisé pour l'analyse est de l'ordre de quelques microlitres. Il est donc très faible par rapport aux quelques millilitres prélevés auprès du patient.

Dans certaines conditions, le prélèvement veineux peut s'avérer inopportun, voire infaisable. Cela peut être notamment le cas pour des enfants, ou pour certaines personnes souffrant de pathologies particulières (personnes obèses, personnes âgées, grands brûlés...). En outre, pour des raisons d'ordre culturel, il est parfois nécessaire de minimiser les volumes prélevés.

Pour répondre à ce type de problématiques, des techniques de micro-prélèvements ont été développées.

On connaît notamment des techniques de prélèvements sanguins issus de vaisseaux capillaires qui permettent de réduire la quantité de sang prélevé et de faciliter l'acte de prélèvement. Le sang des vaisseaux capillaires est prélevé au niveau d'une petite perforation ou d'une petite incision superficielle effectuée par exemple au bout du doigt, ou au niveau de la plante du pied pour les nourrissons, ou encore au niveau du lobe de l'oreille.

Avec ce type de techniques, la quantité de sang prélevé peut être fortement diminuée mais plusieurs approches coexistent.

Dans certains cas, le volume de sang prélevé peut être suffisamment important, par exemple de l'ordre de 200µl, pour pouvoir être récolté dans un tube comme pour le prélèvement veineux classique ou dans un mini-tube. L'échantillon peut donc être directement transféré vers le système d'échantillonnage de l'analyseur sanguin (par exemple du type du MICROS CARE ST®, HORIBA Medical), via l'aiguille de prélèvement, afin d'isoler le volume utilisé pour les analyses. Dans ce cas, l'analyseur fonctionne exactement comme dans le cas d'un échantillon veineux prélevé dans un tube.

Dans d'autres cas, le volume de sang prélevé auprès du patient est très faible, de la taille d'une goutte, c'est-à-dire de l'ordre de 20µl à 50 µl Dans ce cas, le volume de l'échantillon de sang capillaire est trop faible pour être récolté et directement prélevé par le système d'échantillonnage de l'analyseur dans un tube classique. Le volume de sang est alors prélevé à l'aide d'un tube capillaire ou micro-capillaire : l'extrémité du tube capillaire ou micro-capillaire est mise en contact avec la goutte de sang, et le sang emplit le tube par capillarité.

Cependant, en général, le volume de sang prélevé est toujours supérieur au volume effectivement utilisé pour les analyses, ce qui impose encore un échantillonnage de ce volume avant l'analyse.

On connaît notamment le document de brevet FR 2 774 765 qui divulgue un système qui permet de traiter un échantillon de sang prélevé dans un tube micro-capillaire. Le tube capillaire contenant l'échantillon prélevé est introduit dans un conduit fluidique de l'analyseur via un adaptateur. L'échantillon sanguin se retrouve ainsi raccordé à un circuit hydraulique qui permet de diriger l'échantillon vers un premier bac de pré-dilution. Une fois la pré-dilution effectuée, le mélange est dirigé vers le système d'échantillonnage afin de déterminer exactement le volume nécessaire à l'analyse.

La prédilution est rendue indispensable par le fait que la vanne d'échantillonnage utilisée pour l'extraction du volume nécessaire à l'analyse requiert un volume de liquide nettement supérieur au volume du micro-prélèvement.

Le système est donc complexe, il consomme plus de sang que nécessaire pour l'analyse et en outre il implique des manipulations risquées du tube capillaire entre le prélèvement sanguin et son insertion dans un adaptateur.

On connaît également le document JP 4807587 qui divulgue un système permettant de transférer directement le volume de sang prélevé avec un micro-capillaire jusqu'à un système d'échantillonnage qui permet d'isoler le volume nécessaire à l'analyse. Le tube micro-capillaire utilisé pour prélever l'échantillon sanguin est inséré dans un adaptateur. Cet adaptateur est mis en contact manuellement avec l'aiguille de prélèvement de l'analyseur. Le volume nécessaire aux analyses est alors aspiré par l'aiguille.

Un dispositif échantillonneur est également décrit dans le document JP 2008 275353 A.

Toutefois, le prélèvement du sang dans le tube capillaire est dépendant du bon positionnement (sans prise d'air) par l'opérateur du micro-capillaire par rapport à l'aiguille. En effet, si le contact entre le capillaire, via son adaptateur, et l'aiguille n'est pas étanche, le système de prélèvement aspirera de l'air et non pas le sang contenu dans le capillaire.

En outre, les risques de contamination ne sont pas négligeables.

Un objet de la présente invention est de proposer un dispositif d'échantillonnage et de distribution d'échantillons biologiques qui permette de minimiser les volumes d'échantillons prélevés, et d'utiliser au mieux ces volumes prélevés.

Un autre objet de la présente invention est de proposer un dispositif d'échantillonnage et de distribution d'échantillons biologiques qui permette de minimiser les opérations de manipulations d'échantillon et de dilutions.

Un autre objet de la présente invention est de proposer un dispositif d'échantillonnage et de distribution d'échantillons biologiques qui permette de prélever de manière fiable des volumes d'échantillons précis.

Un autre objet de la présente invention est de proposer un dispositif d'échantillonnage et de distribution d'échantillons biologiques qui permette de simplifier les manipulations nécessaires par un opérateur entre le prélèvement de l'échantillon sur un patient et son transfert dans un système d'analyse.

### Exposé de l'invention

Cet objectif est atteint avec un dispositif échantillonneur pour prélever un échantillon de fluide biologique, comprenant:
- un élément capillaire, prevu pour prélever un échantillon de fluide biologique par capillarité,
- une embase solidaire dudit élément capillaire et pourvue d'un premier connecteur prévu pour être fixé de manière étanche et réversible à un second connecteur d'un dispositif de distribution, et pour créer une liaison fluidique entre ledit élément capillaire et ledit dispositif de distribution. distribution (21);
caractérisé en ce que ladite liaison fluidique est réalisée par une ouverture traversante (15), formée dans ladite embase (14), et débouchant dans ledit premier connecteur (16).

Suivant des modes de réalisation, l'élément capillaire et l'embase peuvent être d'un seul tenant, ou réalisés dans une même pièce.

Suivant des modes de réalisation, le dispositif échantillonneur peut comprendre un tube capillaire et une embase pourvue d'un moyen d'accueil apte à recevoir et à maintenir de manière étanche une extrémité dudit tube capillaire.

Dans ce cas, l'élément capillaire, qui est un tube capillaire, et l'embase peuvent alors être des éléments distincts assemblés.

Le dispositif échantillonneur peut comprendre en outre une liaison fluidique qui relie l'élément capillaire ou le moyen d'accueil d'un tube capillaire et le premier connecteur de telle sorte à permettre l'établissement d'une liaison fluidique entre l'élément ou le tube capillaire et le dispositif de distribution.

Ce moyen de liaison fluidique peut en particulier comprendre :
- un canal fluidique, rectiligne ou coudé,
- des parois de l'embase solidaire de l'élément capillaire ou du moyen d'accueil d'un tube capillaire qui s'étendent jusqu'au premier connecteur, par exemple sous la forme d'une ouverture traversant l'embase.

Suivant des modes de réalisation, le dispositif échantillonneur peut comprendre :
- un premier connecteur apte à être verrouillé dans un second connecteur au moyen d'un mouvement comprenant une rotation ;
- un premier connecteur apte à être verrouillé dans un second connecteur au moyen d'un encliquetage ;
- un premier connecteur comprenant un système de verrouillage à baïonnette ;
- un premier connecteur comprenant un filetage ;

L'invention concerne aussi un dispositif de distribution de fluide biologique, qui comprend :
- un second connecteur apte à être fixé de manière étanche et réversible à un premier connecteur d'un dispositif échantillonneur selon l'invention,
- des moyens de transfert de fluide de transfert débouchant dans ledit second connecteur.

Le fluide de transfert peut comprendre par exemple un fluide de dilution ou un réactif.

Le second connecteur peut comprendre en outre des moyens de pression et/ou des moyens de ressort, aptes à exercer une pression sur le premier connecteur lorsqu'il est inséré dans le second connecteur afin d'assurer l'étanchéité de la liaison.

Les moyens de transfert de fluide de transfert peuvent notamment comprendre une conduite de fluide et/ou un tube.

Suivant des modes de réalisation, le dispositif de distribution peut comprendre en outre des moyens d'injection aptes à pousser un volume prédéfini de fluide de transfert au travers des moyens de transfert de fluide de transfert en direction du second connecteur.

Les moyens d'injection peuvent comprendre une seringue.

Suivant des modes de réalisation, le dispositif de distribution peut comprendre en outre un bac de mesure disposé de telle sorte à pouvoir recevoir directement du fluide issu d'un élément capillaire d'un dispositif échantillonneur fixé sur le second connecteur.

Suivant des modes de réalisation, le dispositif de distribution peut comprendre en outre des moyens de déplacement aptes à positionner le second connecteur :
- selon une première position, de telle sorte à permettre la fixation et le retrait d'un dispositif échantillonneur dans ledit second connecteur,
- selon une seconde position, de telle sorte qu'un fluide de transfert issu d'un dispositif échantillonneur inséré dans ledit second connecteur puisse s'écouler dans le bac de mesure.

Les moyens de déplacement peuvent comprendre :
- des moyens de rotation ;
- des moyens de translation.

Suivant un autre mode de réalisation de l'invention, il est proposé un dispositif de distribution miniaturisé pouvant comprendre un bac de mesure et un premier récipient.

Avantageusement, le dispositif de distribution portatif selon ce mode de réalisation alternatif peut comprendre des moyens d'injection aptes à pousser le liquide contenu dans ledit premier récipient, exclusivement au travers de la conduite de fluide et de l'élément capillaire. De cette façon, le risque de contamination du prélèvement sanguin est minime.

Avantageusement, le bac de mesure selon ce mode de réalisation alternatif, peut comprendre par ailleurs des moyens d'analyse optique et/ou électrique du liquide qu'il contient.

Suivant une autre alternative de ce mode de réalisation, le dispositif de distribution miniaturisé peut posséder en outre un moyen d'interfaçage apte à connecter au moins mécaniquement le dispositif de distribution miniaturisé avec un autre dispositif d'analyse.

Avantageusement, le moyen d'interfaçage selon ce mode de réalisation alternatif peut comprendre en outre une liaison fluidique.

Suivant un autre aspect, il est proposé un dispositif d'échantillonnage et de distribution de fluide biologique, qui comprend un dispositif de distribution selon l'invention et au moins un dispositif échantillonneur selon l'invention.

Suivant un autre aspect, il est proposé un appareil d'analyse de fluides biologiques, qui comprend un dispositif de distribution de fluide biologique selon l'invention.

L'appareil d'analyse de fluides biologiques peut comprendre en outre au moins un dispositif échantillonneur selon l'invention.

De manière générale, les fluides ou liquides biologiques pour lesquels l'invention peut être mise en œuvre peuvent comprendre des fluides corporels tels que du sang, du sérum, du plasma, de la salive, de l'urine, du liquide céphalo-rachidien ou encore des extraits tissulaires comme de la moelle osseuse.

Suivant des modes de réalisation, l'appareil selon l'invention peut être destiné à analyser un fluide biologique comprenant du sang.

Suivant des modes de réalisation, l'appareil selon l'invention peut comprendre en outre une aiguille de prélèvement et des moyens de dérivation permettant de transférer du fluide de transfert, soit vers ladite aiguille de prélèvement, soit vers le dispositif de distribution.

L'aiguille de prélèvement permet de prélever un fluide biologique dans un tube ouvert ou fermé classique. Ainsi, l'appareil peut être adapté à la mesure d'échantillons conditionnés soit dans des tubes classiques soit dans des tubes capillaires.

Suivant un autre aspect, il est proposé un procédé d'échantillonnage et de distribution d'un fluide biologique, mettant en œuvre un dispositif échantillonneur pourvu d'un élément capillaire et un dispositif de distribution, lequel procédé comprenant une étape de transfert et de dilution d'un échantillon de fluide biologique contenu dans ledit élément capillaire en injectant un volume prédéfini de fluide de transfert au travers des moyens de transfert de fluide de transfert et dudit élément capillaire.

Suivant des modes de mise en œuvre, le procédé d'échantillonnage et de distribution selon l'invention peut comprendre :
- une étape de transfert de l'échantillon de fluide biologique et d'un volume de fluide de transfert déterminé dans un bac de mesure, de telle sorte à réaliser dans ledit bac de mesure une dilution du fluide biologique directement adaptée à une opération d'analyse biologique;
- une étape préalable de prélèvement d'un échantillon de fluide biologique par capillarité dans un élément capillaire d'un dispositif échantillonneur selon l'invention.

Suivant des modes de mise en œuvre, le procédé d'échantillonnage et de distribution selon l'invention peut être mis en œuvre avec un fluide biologique comprenant du sang.

Il peut comprendre une étape de transfert d'un volume défini de fluide de transfert adapté pour réaliser une dilution directement adaptée à au moins l'une des opérations d'analyse biologique suivantes :
- un comptage de cellules (telles que des globules rouges, des globules blancs, des cellules immatures...),
- un comptage d'éléments figurés,
- un dosage d'un analyte présent dans le fluide biologique,
- une caractérisation cellulaire.

### Description des figures et modes de réalisation

D'autres avantages et particularités de l'invention apparaîtront à la lecture de la description détaillée de mises en œuvre et de modes de réalisation nullement limitatifs, et des dessins annexés suivants :
- la Fig. 1 illustre un premier mode de réalisation de dispositif échantillonneur selon l'invention,
- la Fig. 2 illustre un second mode de réalisation de dispositif échantillonneur selon l'invention, avec Fig. 2(a) une vue en perspective et Fig. 2(b) une vue en coupe,
- la Fig. 3 illustre un dispositif échantillonneur et un dispositif de distribution selon l'invention, déconnectés l'un de l'autre,
- la Fig. 4 illustre un dispositif échantillonneur et un dispositif de distribution selon l'invention, fixés l'un à l'autre,
- la Fig. 5 illustre un appareil selon l'invention, avec le dispositif de distribution dans une position permettant la fixation et le retrait d'un dispositif échantillonneur,
- la Fig. 6 illustre un appareil selon l'invention, avec le dispositif de distribution dans une position avec le dispositif échantillonneur en regard du bac de mesure.
- la Fig. 7 illustre un dispositif de distribution miniaturisé selon un autre mode de réalisation de l'invention, avec un dispositif échantillonneur à proximité.
- la Fig. 8 illustre un dispositif échantillonneur couplé à un dispositif de distribution miniaturisé selon l'invention.
- la Fig. 9 illustre un dispositif de distribution miniaturisé selon l'invention dans lequel le dispositif d'échantillonnage est en cours de déplacement.
- la Fig. 10 illustre le dispositif de distribution miniaturisé selon l'invention contenant le dispositif échantillonneur dans sa position de distribution.
- la Fig. 11 illustre une vue de face en semi-transparence selon la fig. 10.

On va maintenant décrire des modes de réalisation de dispositifs selon l'invention destinés à être mis en œuvre dans des appareils d'analyse sanguine.

Comme expliqué précédemment, l'invention comprend pour l'essentiel un dispositif échantillonneur et un dispositif de distribution apte à recevoir le dispositif échantillonneur.

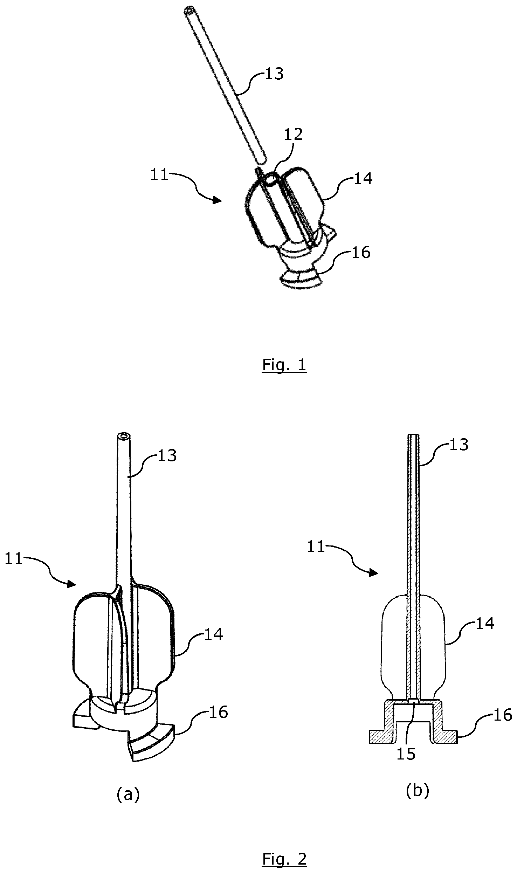

En référence aux Fig. 1 et Fig. 2, nous allons dans un premier temps décrire deux modes de réalisation (équivalents sur le plan fonctionnel) du dispositif échantillonneur 11.

Selon un premier mode de réalisation présenté à la Fig. 1, le dispositif échantillonneur 11 comprend un tube micro-capillaire 13 ou tube capillaire 13. Ce tube capillaire 13 comprend un conduit interne dimensionné de telle sorte qu'un fluide biologique (tel que du sang) puisse s'y introduire par capillarité de telle sorte à le remplir.

Le tube capillaire 13 est de préférence réalisé dans un matériau transparent qui permet de vérifier la présence de sang à l'intérieur. Il peut être par exemple réalisé en verre ou en matière plastique. Les parois intérieures du tube capillaire peuvent être traitées spécifiquement en fonction du fluide biologique prélevé. Par exemple un anticoagulant peut être déposé dans le cas du prélèvement d'un échantillon de sang.

Le dispositif échantillonneur 11 comprend également une embase 14. Cette embase 14 comprend un moyen d'accueil 12 ou un réceptacle 12 destiné à recevoir l'extrémité du tube capillaire 13, de telle sorte à le maintenir et à assurer l'étanchéité de la liaison.

L'embase 14 comprend également un premier connecteur 16 destiné à fixer le dispositif échantillonneur 11, de manière étanche et réversible, sur un second connecteur 22 d'un dispositif de distribution 21, comme cela sera détaillé plus loin.

L'embase 14 comprend aussi une liaison fluidique 15, sous la forme d'une ouverture traversante débouchant dans le premier connecteur 16, et située dans le prolongement du réceptacle 12 et du tube capillaire 13.

L'embase 14 peut être par exemple réalisée en matériau plastique ou polymère moulé et/ou usiné. Elle peut être pourvue de reliefs ou d'ailettes comme illustré à la Fig. 1 pour faciliter sa préhension et sa manipulation.

Selon un second mode de réalisation présenté à la Fig. 2, le dispositif échantillonneur 11 comprend un élément capillaire 13 et une embase 14 qui sont réalisées dans une même pièce (ou en un seul bloc).

L'élément capillaire 13 comprend un conduit interne dimensionné de telle sorte qu'un fluide biologique (tel que du sang) puisse s'y introduire par capillarité de telle sorte à le remplir.

L'embase 14 comprend également un premier connecteur 16 destiné à fixer le dispositif échantillonneur 11, de manière étanche et réversible, sur un second connecteur 22 d'un dispositif de distribution 21.

L'embase 14 comprend aussi une liaison fluidique 15, sous la forme d'une ouverture traversante débouchant dans le premier connecteur 16, et située dans le prolongement du conduit interne de l'élément capillaire 13.

L'élément capillaire 13 et l'embase 14 peuvent être par exemple réalisés dans une pièce en matériau plastique ou polymère moulé et/ou usiné.

Cette pièce peut être pourvue de reliefs ou d'ailettes au niveau de l'embase 14 comme illustré à la Fig. 2 pour faciliter sa préhension et sa manipulation.

L'élément capillaire 13 peut être de toute forme à l'extérieur. Dans le mode de réalisation présenté à la Fig. 2, sa forme externe est celle d'un tube. Aussi, sans perte de généralité, il sera également appelé dans la suite « tube capillaire 13 ».

Dans les deux modes de réalisation présentés, le dispositif échantillonneur 11 avec son tube capillaire 13 constitue un dispositif de micro-prélèvement de fluide biologique directement utilisable pour effectuer par exemple une ponction capillaire. En effet, lorsque l'extrémité du tube capillaire 13 est mise en contact avec une goutte de fluide biologique (tel que du sang), le fluide biologique s'élève par capillarité dans le tube 13 jusqu'à le remplir.

Suivant un aspect avantageux de l'invention, le tube capillaire 13 est dimensionné de telle sorte que son volume intérieur définisse un volume d'échantillon précis, par exemple 10 microlitres.

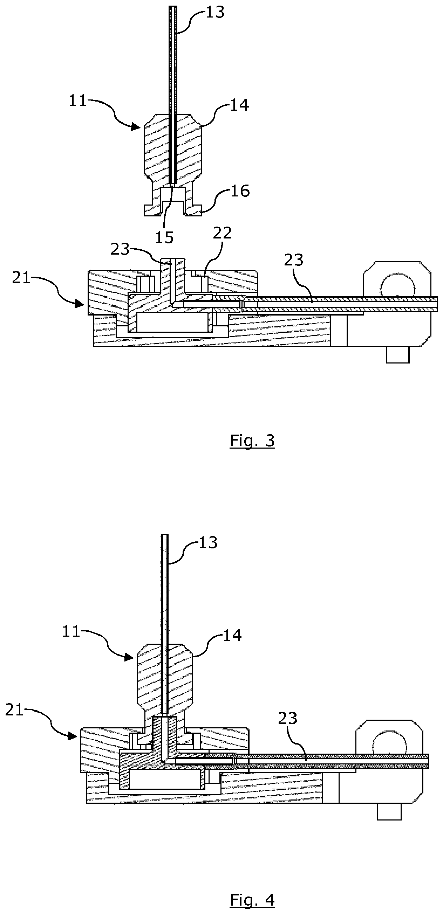

En référence aux Fig. 3 et Fig. 4, nous allons maintenant décrire le dispositif de distribution 21 selon l'invention.

Ce dispositif de distribution 21 correspond à l'interface d'entrée d'un analyseur sanguin. Il a pour fonction de recevoir des dispositifs échantillonneur 11 (réalisés par exemple selon l'un ou l'autre deux modes de réalisation présentés) avec des prélèvements sanguins, et de transférer ces prélèvements dans des moyens d'analyse de l'analyseur sanguin.

Il comprend un second connecteur 22 destiné à recevoir le premier connecteur 16 du dispositif échantillonneur 11 comme expliqué précédemment.

Le premier connecteur 16 comprend des ergots prévus pour s'engager dans des logements du second connecteur 22, de telle sorte que par une rotation d'un quart de tour le dispositif échantillonneur 11 puisse être fixé sur le dispositif de distribution 21 comme illustré à la Fig. 3. Un système de ressorts (non représenté sur les figures) assure le maintien d'une pression constante pour garantir l'étanchéité de la liaison.

Le dispositif de distribution 21 comprend également une conduite de fluide 23 qui débouche dans le second connecteur 22, de telle sorte à établir une liaison fluidique avec le tube capillaire 13 au travers de l'embase 14 lorsqu'un dispositif échantillonneur 11 est connecté. Avantageusement, le tube capillaire 13 n'effleure pas la conduite de fluide 23 lorsque l'embase 14 est fixée sur le connecteur 22, grâce à la présence d'un espace d'air dans la liaison fluidique 15.

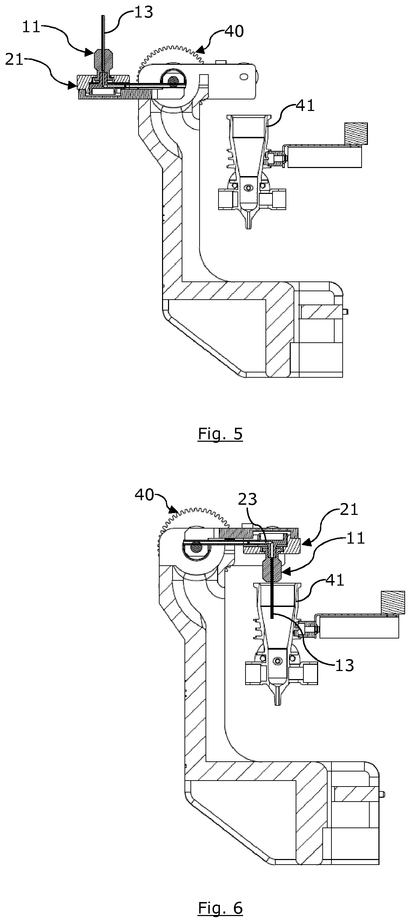

En référence aux Fig. 5 et Fig. 6, le dispositif de distribution 21 comprend également une cuve de mesure 41.

Suivant un aspect avantageux de l'invention, cette cuve de mesure 41 est destinée à recevoir directement l'échantillon sanguin prélevé dans le tube capillaire 13 et un volume de liquide de dilution qui permet d'obtenir dans cette cuve 41 la dilution ou le mélange nécessaire aux opérations de mesure : par exemple une dilution de 1/300 pour un comptage de cellules blanches du sang ou une dilution de 1/15000 pour un comptage de cellules rouges du sang.

Pour effectuer ces mesures, la cuve de mesure 41 est reliée par exemple à un dispositif de mesures optiques ou résistives ou de mesure de cytométrie en flux, selon des techniques bien connues de l'homme du métier.

Pour transférer l'échantillon de sang et le liquide de dilution dans la cuve de mesure 41, le tube capillaire est positionné en regard de cette cuve de mesure 41 selon la configuration de la Fig. 6.

Du liquide de dilution est injecté dans le conduit 23 du dispositif de distribution 21, en direction du tube capillaire 13. Ce liquide de dilution expulse dans un premier temps l'échantillon de sang du tube capillaire 13 dans la cuve de mesure 41, puis s'écoule au travers du tube capillaire 13 dans cette cuve de mesure 41.

Un système de dosage, par exemple une seringue, est utilisé pour injecter ainsi une quantité prédéfinie de liquide de dilution dans le conduit 23 et donc dans la cuve de mesure 41, de telle sorte à atteindre un taux de dilution précis dans cette cuve de mesure 41 (connaissant le volume d'échantillon de sang contenu dans le tube capillaire 13).

L'homogénéité de la solution dans la cuve de mesure 41 est assurée par un dispositif d'agitation par exemple par circulations de bulles de gaz (bullage).

Ainsi, suivant des aspects avantageux de l'invention :
- l'échantillon sanguin est transféré directement du tube capillaire 13 qui a servi à son prélèvement dans la cuve de mesure 41, sans transiter par une conduite interne du système. Les risques de contamination sont ainsi minimisés ;
- la totalité du prélèvement sanguin peut être effectivement utilisée pour les mesures, sans pertes. En effet, on peut compter toutes les cellules de l'échantillon sanguin en faisant par exemple transiter en totalité la solution diluée dans la cuve de mesure 41 dans le système d'analyse. Il est ainsi possible de prélever et d'utiliser de très faibles quantités de sang ;
- le contrôle de la dilution peut être très précis, car on est sûr de transférer la totalité de l'échantillon contenu dans le tube capillaire 13 dans la cuve de mesure 41 ;
- la manipulation des échantillons par un opérateur est aisée et sans risque car le dispositif échantillonneur 11 peut être utilisé directement pour effectuer le prélèvement sur une personne, puis inséré sur le connecteur 22 du dispositif de distribution 21. Les manipulations sont ainsi minimisées et simplifiées au maximum. En outre, le tube capillaire 13 et son embase 14 sont positionnés et bloqués sur le dispositif de distribution 21, et de ce fait la connexion avec l'instrument est indépendante de l'habileté d'un opérateur. En effet, l'embase 14 est centrée sur le dispositif de distribution 21 et est maintenue en contact par le système à ressort et le verrouillage quart de tour de manière à assurer une pression constante et une bonne étanchéité entre le capillaire 13, via son embase 14, et le système de distribution 21.

Il est à noter d'ailleurs que des dispositifs échantillonneurs 11 peuvent être fournis sous forme de dispositifs de prélèvement à usage unique, amenés à l'analyseur sanguin après le prélèvement pour effectuer les mesures.

Afin de faciliter les manipulations, le dispositif de distribution 21 comprend des moyens de déplacement 40 sous la forme d'un système rotatif 40 comme illustré aux Fig. 5 et Fig. 6. Le déplacement peut être manuel ou motorisé.

Le dispositif de distribution 21 peut ainsi se déplacer entre deux positions :
- une position de chargement, illustrée Fig. 5, adaptée à la mise en place et au retrait du dispositif échantillonneur 11 sur le connecteur 22 du dispositif de distribution 21, et
- une position de distribution, illustrée Fig. 6, dans laquelle le dispositif échantillonneur 11 est positionné en regard ou à l'aplomb de la cuve de mesure 41.

Dans la mesure où le mouvement de rotation a lieu dans un plan perpendiculaire à la façade de l'analyseur sanguin, la position de chargement correspond à une position externe à l'appareil, tandis que la position de distribution correspond à une position interne à l'appareil.

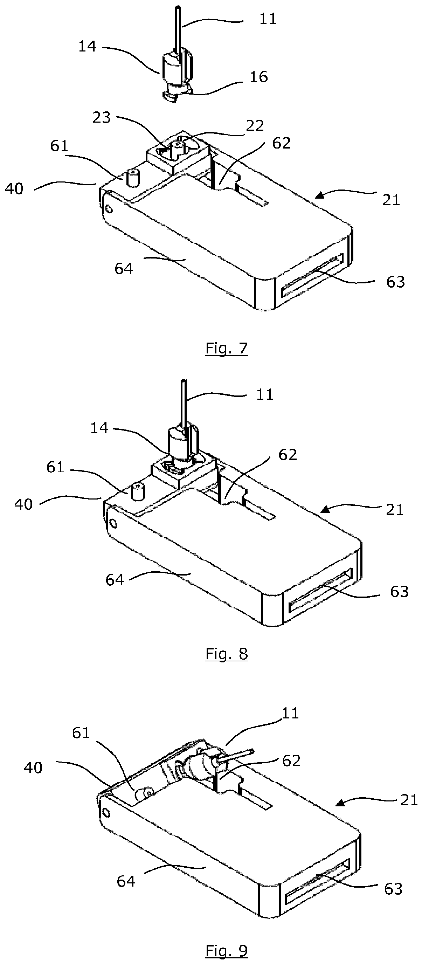

En référence aux figures 7 à 11, il est décrit un autre mode de réalisation de la présente invention. Ce mode de réalisation particulier répond à une problématique d'amélioration des diagnostics médicaux et de la prise en charge des patients. Il s'agit d'une approche dite « point-of-care » pour laquelle les prélèvements et analyses sont réalisés et interprétés au plus proche du patient plutôt que dans un laboratoire central afin de prendre une décision clinique le plus rapidement possible. Pour ce faire, il est indispensable de pouvoir prélever, conditionner et transporter des échantillons sanguins de manière sûre, efficace et durable. Le mode de réalisation particulier de la présente invention répond à ce problème technique et consiste en un dispositif de distribution 21 portatif apte d'une part à recevoir un dispositif échantillonneur 11 avec des prélèvements sanguins et d'autre part à transférer lesdits prélèvements dans une cuve de mesure 41.

On entend par dispositif de distribution portatif un dispositif apte à être facilement manipulé et transporté par un opérateur. Par exemple, les dimensions extérieures peuvent être légèrement supérieures à celles du dispositif échantillonneur afin de pouvoir le contenir entièrement. A titre indicatif, on peut par exemple considérer des dimensions de l'ordre de 5x8x1 cm, pour un poids de l'ordre la centaine de grammes. Avantageusement, les dimensions du dispositif de distribution portatif sont telles qu'il tient facilement dans la main. Ces grandeurs sont données à titre d'exemple afin d'illustrer la transportabilité de ce mode de réalisation de l'invention et ne constituent pas en tant que telles une limitation de la portée revendiquée de l'invention.

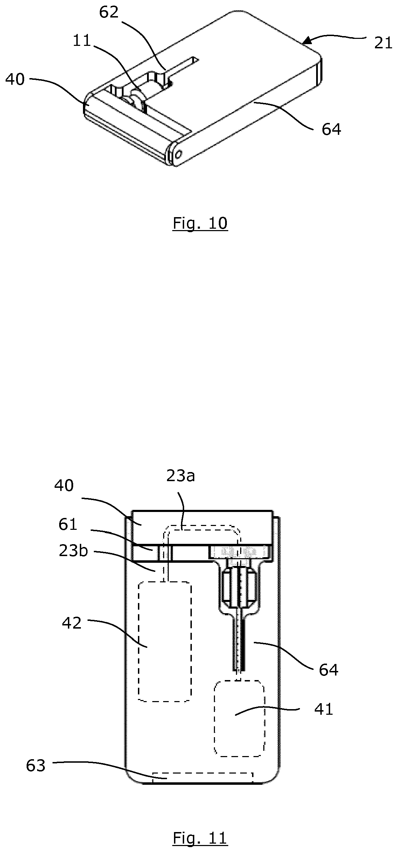

Le dispositif de distribution portatif 21 comprend notamment :
- des seconds connecteurs 22 destinés à recevoir les premiers connecteurs 16 du dispositif échantillonneur 11 afin de créer une liaison étanche et réversible entre le dispositif échantillonneur 11 et le dispositif de distribution portatif 21,
- une conduite de fluide 23 débouchant dans les seconds connecteurs 22 permettant d'établir une liaison fluidique avec le tube capillaire 13 au travers de l'embase 14 du dispositif échantillonneur 11. La conduite fluidique 23 comprend une première partie 23a située au travers les moyens de déplacements 40 et une deuxième partie 23b située dans le corps 64 du dispositif de distribution portatif 21,
- des moyens de déplacement 40 aptes à positionner le connecteur dans une première position - dite de chargement - pour laquelle il est possible de fixer ou de retirer le dispositif échantillonneur 11 (figure 8) ; et dans une seconde position - dite de distribution - pour laquelle une liaison fluidique est établie entre le dispositif de distribution 21 portatif et le dispositif échantillonneur 11 (figure 10),
- un connecteur fluidique 61,
- un premier récipient 42,
- un second récipient 41.

Selon ce mode de réalisation alternatif, le dispositif de distribution portatif 21 possède en outre un logement 62 dans le corps 64. Le logement 62 est apte à accueillir au moins une partie du dispositif échantillonneur 11. Lorsque les moyens de déplacement 40 sont actionnés - manuellement ou automatiquement - le dispositif échantillonneur est déplacé jusque dans ledit logement 62. Dans le cas illustré sur les figures 8 à 10, les moyens de déplacement 40 consistent en des moyens de rotation.

Lorsque le dispositif de distribution portatif 21 est dans la position de distribution illustrée sur les figures 10 et 11, le dispositif échantillonneur 11 est connecté de manière étanche à la conduite de fluide 23 du dispositif de distribution portatif 21. Le connecteur fluidique 61 assure la liaison entre la portion de la conduite fluidique 23a qui est comprise dans les moyens de déplacement 40 et celle 23b qui est intégrée dans le corps 64 du dispositif de distribution portatif 21. La liaison est assurée de manière étanche au moins lorsque le dispositif de distribution portatif 21 est dans la position de distribution. Le connecteur fluidique peut prendre n'importe quelle forme sans restriction de la présente invention. Dans l'exemple illustré sur les figures 7 à 10, le connecteur fluidique 61 prend la forme d'un ergot à l'intérieur duquel débouche la conduite fluidique 23. Suivant un autre mode de réalisation, le connecteur fluidique peut prendre la forme d'un joint tournant, situé à l'interface entre les moyens de déplacement 40 et le corps 64 du dispositif de distribution portatif 21.

Par ailleurs, dans cette position de distribution, le dispositif échantillonneur 11 est au moins partiellement logé dans le corps 64 du dispositif de distribution miniaturisé 21 afin de le protéger par exemple du milieu extérieur et d'éventuels dommages durant les analyses.

Le dispositif de distribution miniaturisé 21 possède en outre un moyen d'interfaçage 63 qui permet de connecter au moins mécaniquement le dispositif de distribution miniaturisé 21 avec un autre dispositif d'analyse. Selon un autre mode de réalisation, cette connexion peut être électronique et/ou inclure une liaison fluidique.

La figure 11 illustre une vue en semi-transparence du dispositif de distribution miniaturisé 21 lorsque le dispositif échantillonneur est en position de distribution. La conduite fluidique 23 permet ainsi de connecter le dispositif échantillonneur 11 à d'une part un premier récipient 42 et d'autre part à un second récipient 41.

Le premier récipient 42 peut contenir par exemple un diluant ou un liquide de transfert ; il peut inclure à cet effet des moyens d'injection afin de faire circuler un fluide au travers de la conduite fluidique 23.

Le second récipient 41 consiste en un bac de mesure dans lequel sont déversés le mélange formé par le liquide contenu dans le premier récipient 42 et le prélèvement sanguin contenu dans le capillaire 13 du dispositif échantillonneur 11. Le second récipient peut inclure des moyens de mesure et/ou d'analyse du mélange ainsi réalisé.

Bien sûr, l'invention n'est pas limitée aux exemples qui viennent d'être décrits et de nombreux aménagements peuvent être apportés à ces exemples sans sortir du cadre de l'invention.

## Revendications

1. Dispositif échantillonneur (11) pour prélever un échantillon de fluide biologique, comprenant:
- un élément capillaire (13) prévu pour prélever un échantillon de fluide biologique par capillarité, et
- une embase (14) solidaire dudit élément capillaire (13) et pourvue d'un premier connecteur (16) prévu pour être fixé de manière étanche et réversible à un second connecteur (22) d'un dispositif de distribution (21) et pour créer une liaison fluidique entre ledit élément capillaire (13) et ledit dispositif de distribution (21) ;
**caractérisé en ce que** ladite liaison fluidique est réalisée par une ouverture traversante (15), formée dans ladite embase (14), et débouchant dans ledit premier connecteur (16).

2. Dispositif échantillonneur selon la revendication 1, comprenant un tube capillaire (13) et une embase (14) pourvue d'un moyen d'accueil (12) apte à recevoir et à maintenir de manière étanche une extrémité dudit tube capillaire (13).

3. Dispositif échantillonneur selon l'une des revendications 1 ou 2, comprenant un premier connecteur (16) apte à être verrouillé dans un second connecteur (22) au moyen d'un mouvement comprenant une rotation.

4. Dispositif de distribution (21) de fluide biologique, **caractérisé en ce qu'**il comprend :
- un second connecteur (22) apte à être fixé de manière étanche et réversible à un premier connecteur (16) d'un dispositif échantillonneur (11) selon l'une quelconque des revendications précédentes,
- des moyens de transfert de fluide de transfert (23) débouchant dans ledit second connecteur (22).

5. Dispositif de distribution selon la revendication 4, comprenant en outre des moyens d'injection aptes à pousser un volume prédéfini de fluide de transfert au travers des moyens de transfert de fluide de transfert (23) en direction du second connecteur (22).

6. Dispositif de distribution selon la revendication 5, dans lequel les moyens d'injection comprennent une seringue.

7. Dispositif de distribution selon l'une des revendications 4 à 6, comprenant en outre un bac de mesure (41) disposé de telle sorte à pouvoir recevoir directement du fluide issu d'un élément capillaire (13) d'un dispositif échantillonneur (11) fixé sur le second connecteur (22).

8. Dispositif de distribution de la revendication 7, comprenant en outre des moyens de déplacement (40) aptes à positionner le second connecteur (22) :
- selon une première position, de telle sorte à permettre la fixation et le retrait d'un dispositif échantillonneur (11) dans ledit second connecteur (22),
- selon une seconde position, de telle sorte qu'un fluide de transfert issu d'un dispositif échantillonneur (11) inséré dans ledit second connecteur (22) puisse s'écouler dans le bac de mesure (41).

9. Dispositif de distribution selon la revendication 8, dans lequel les moyens de déplacement (40) comprennent des moyens de rotation.

10. Dispositif de distribution (21) selon l'une quelconque des revendications 4 à 9, comprenant un bac de mesure (41) et un premier récipient (42), ledit dispositif de distribution (21) étant portatif.

11. Dispositif de distribution (21) portatif selon la revendication précédente, **caractérisé en ce que** le premier récipient (42) comprend des moyens d'injection aptes à pousser le liquide contenu dans ledit premier récipient (42), exclusivement au travers de la conduite de fluide 23 et de l'élément capillaire (13).

12. Dispositif de distribution (21) portatif selon l'une quelconque des revendications 10 ou 11, **caractérisé en ce qu'**il comprend en outre un moyen d'interfaçage (63) apte à connecter au moins mécaniquement ledit dispositif de distribution (21) portatif avec un autre dispositif d'analyse.

13. Dispositif de distribution (21) portatif selon la revendication précédente, **caractérisé en ce que** le moyen d'interfaçage (63) comprend en outre une liaison fluidique.

14. Appareil d'analyse de fluides biologiques, **caractérisé en ce qu'**il comprend un dispositif de distribution de fluide biologique selon l'une des revendications 4 à 13.

15. Appareil selon la revendication 14, destiné à analyser un fluide biologique comprenant du sang.

16. Procédé d'échantillonnage et de distribution d'un fluide biologique, mettant en œuvre un dispositif échantillonneur (11) selon l'une des revendications 2 ou 3 pourvu d'un élément capillaire (13) et un dispositif de distribution (21) selon l'une des revendications 4 à 13, **caractérisé en ce qu'**il comprend une étape de transfert et de dilution d'un échantillon de fluide biologique contenu dans ledit élément capillaire (13) en injectant un volume prédéfini de fluide de transfert au travers des moyens de transfert de fluide de transfert (23) et dudit élément capillaire (13).

17. Procédé d'échantillonnage et de distribution selon la revendication 16, comprenant une étape de transfert de l'échantillon de fluide biologique et d'un volume de fluide de transfert déterminé dans un bac de mesure (41), de telle sorte à réaliser dans ledit bac de mesure (41) une dilution du fluide biologique directement adaptée à une opération d'analyse biologique.

18. Procédé d'échantillonnage et de distribution de l'une des revendications 16 ou 17, comprenant une étape préalable de prélèvement d'un échantillon de fluide biologique par capillarité dans un élément capillaire (13) d'un dispositif échantillonneur (11).

19. Procédé d'échantillonnage et de distribution selon l'une des revendications 16 à 18, mis en œuvre avec un fluide biologique comprenant du sang.

20. Procédé d'échantillonnage et de distribution selon la revendication 19, comprenant une étape de transfert d'un volume défini de fluide de transfert adapté pour réaliser une dilution directement adaptée à au moins l'une des opérations d'analyse biologique suivantes :
- un comptage de cellules,
- un comptage d'éléments figurés,
- un dosage d'un analyte présent dans le fluide biologique,
- une caractérisation cellulaire.

## Patentansprüche

1. Probenahmevorrichtung (11) zum Entnehmen einer Probe eines biologischen Fluids, die Folgendes beinhaltet:
- ein Kapillarelement (13), das dazu vorgesehen ist, eine Probe eines biologischen Fluids durch Kapillarwirkung zu entnehmen, und
- ein Unterteil (14), das mit dem Kapillarelement (13) fest verbunden ist und über ein erstes Verbindungselement (16) verfügt, das dazu vorgesehen ist, dichtend und reversibel an einem zweiten Verbindungselement (22) einer Abgabevorrichtung (21) befestigt zu werden und eine Fluidverbindung zwischen dem Kapillarelement (13) und der Abgabevorrichtung (21) herzustellen;
**dadurch gekennzeichnet, dass** die Fluidverbindung durch eine Durchgangsöffnung (15) erfolgt, die in dem Unterteil (14) gebildet ist und in dem ersten Verbindungselement (16) mündet.

2. Probenahmevorrichtung nach Anspruch 1, beinhaltend ein Kapillarrohr (13) und ein Unterteil (14), das über ein Aufnahmemittel (12) verfügt, das fähig ist, ein Ende des Kapillarrohrs (13) aufzunehmen und dichtend zu halten.

3. Probenahmevorrichtung nach einem der Ansprüche 1 oder 2, beinhaltend ein erstes Verbindungselement (16), das fähig ist, mittels einer Bewegung, die eine Drehung beinhaltet, in einem zweiten Verbindungselement (22) verriegelt zu werden.

4. Vorrichtung (21) für die Abgabe eines biologischen Fluids, **dadurch gekennzeichnet, dass** sie Folgendes beinhaltet:
- ein zweites Verbindungselement (22), das fähig ist, dichtend und reversibel an einem ersten Verbindungselement (16) einer Probenahmevorrichtung (11) nach einem der vorhergehenden Ansprüche befestigt zu werden,
- Mittel für den Transfer eines Transferfluids (23), die in dem zweiten Verbindungselement (22) münden.

5. Abgabevorrichtung nach Anspruch 4, ferner beinhaltend Injektionsmittel, die fähig sind, ein vordefiniertes Transferfluidvolumen durch Transferfluid-Transfermittel (23) in Richtung des zweiten Verbindungselements (22) zu drücken.

6. Abgabevorrichtung nach Anspruch 5, wobei die Injektionsmittel eine Spritze beinhalten.

7. Abgabevorrichtung nach einem der Ansprüche 4 bis 6, ferner beinhaltend eine Messwanne (41), die so angeordnet ist, dass sie Fluid, das aus einem Kapillarelement (13) einer an dem zweiten Verbindungselement (22) befestigten Probenahmevorrichtung (11) stammt, direkt aufnehmen kann.

8. Abgabevorrichtung nach Anspruch 7, ferner beinhaltend Bewegungsmittel (40), die fähig sind, das zweite Verbindungselement (22) folgendermaßen zu positionieren:
- in einer ersten Position, in der das Befestigen einer Probenahmevorrichtung (11) in dem zweiten Verbindungselement (22) und ihr Entfernen daraus möglich ist,
- in einer zweiten Position, in der ein Transferfluid, das aus einer in das zweite Verbindungselement (22) eingeführten Probenahmevorrichtung (11) stammt, in die Messwanne (41) fließen kann.

9. Abgabevorrichtung nach Anspruch 8, wobei die Bewegungsmittel (40) Drehmittel beinhalten.

10. Abgabevorrichtung (21) nach einem der Ansprüche 4 bis 9, beinhaltend eine Messwanne (41) und einen ersten Behälter (42), wobei die Abgabevorrichtung (21) tragbar ist.

11. Tragbare Abgabevorrichtung (21) nach dem vorhergehenden Anspruch, **dadurch gekennzeichnet, dass** der erste Behälter (42) Injektionsmittel beinhaltet, die fähig sind, die in dem ersten Behälter (42) enthaltene Flüssigkeit ausschließlich durch die Fluidleitung 23 und das Kapillarelement (13) zu drücken.

12. Tragbare Abgabevorrichtung (21) nach einem der Ansprüche 10 oder 11, **dadurch gekennzeichnet, dass** sie ferner ein Schnittstellenmittel (63) beinhaltet, das fähig ist, die tragbare Abgabevorrichtung (21) mindestens mechanisch mit einer anderen Analysevorrichtung zu verbinden.

13. Tragbare Abgabevorrichtung (21) nach dem vorhergehenden Anspruch, **dadurch gekennzeichnet, dass** das Schnittstellenmittel (63) ferner eine Fluidverbindung beinhaltet.

14. Gerät für die Analyse von biologischen Fluiden, **dadurch gekennzeichnet, dass** es eine Vorrichtung für die Abgabe eines biologischen Fluids nach einem der Ansprüche 4 bis 13 beinhaltet.

15. Gerät nach Anspruch 14, das dazu bestimmt ist, ein biologisches Fluid, das Blut beinhaltet, zu analysieren.

16. Verfahren zur Probenahme und Abgabe eines biologischen Fluids, das eine Probenahmevorrichtung (11) nach einem der Ansprüche 2 oder 3, die über ein Kapillarelement (13) verfügt, und eine Abgabevorrichtung (21) nach einem der Ansprüche 4 bis 13 verwendet, **dadurch gekennzeichnet, dass** es einen Schritt des Transfers und des Verdünnens einer Probe eines biologischen Fluids, die in dem Kapillarelement (13) enthalten ist, beinhaltet, indem ein vordefiniertes Transferfluidvolumen durch Transferfluid-Transfermittel (23) und das Kapillarelement (13) injiziert wird.

17. Probenahme- und Abgabeverfahren nach Anspruch 16, beinhaltend einen Schritt des Transfers der Probe eines biologischen Fluids und eines bestimmten Transferfluidvolumens in eine Messwanne (41), derart, dass in der Messwanne (41) eine Verdünnung des biologischen Fluids durchgeführt wird, die für einen biologischen Analysevorgang direkt geeignet ist.

18. Probenahme- und Abgabeverfahren nach einem der Ansprüche 16 oder 17, beinhaltend einen vorausgehenden Schritt des Entnehmens einer Probe eines biologischen Fluids durch Kapillarwirkung in einem Kapillarelement (13) einer Probenahmevorrichtung (11).

19. Probenahme- und Abgabeverfahren nach einem der Ansprüche 16 bis 18, das mit einem biologischen Fluid, das Blut beinhaltet, verwendet wird.

20. Probenahme- und Abgabeverfahren nach Anspruch 19, beinhaltend einen Schritt des Transfers eines definierten Transferfluidvolumens, das dazu ausgelegt ist, eine Verdünnung, die für mindestens einen der folgenden biologischen Analysevorgänge direkt geeignet ist, durchzuführen:
- eine Zählung von Zellen,
- eine Zählung von Bestandteilen,
- eine Dosierung eines Analyten, der in dem biologischen Fluid vorhanden ist,
- eine Zellcharakterisierung.

## Claims

1. Sampler device (11) for collecting a sample of biological fluid, comprising:
- a capillary component (13) provided for collecting a sample of biological fluid by capillary action, and
- a base (14) rigidly connected to said capillary component (13) and provided with a first connector (16) provided for being reversibly attached in a leaktight manner to a second connector (22) of a dispensing device (21) and for creating a fluid connection between said capillary component (13) and said dispensing device (21);
**characterized in that** said fluid connection is made by a continuous opening (15) which is formed in said base (14) and opens into said first connector (16).

2. Sampler device according to Claim 1, comprising a capillary tube (13) and a base (14) which is provided with a receiving means (12) capable of receiving an end of said capillary tube (13) and of keeping same leaktight.

3. Sampler device according to either of Claims 1 and 2, comprising a first connector (16) capable of being locked in a second connector (22) by means of a movement comprising a rotation.

4. Dispensing device (21) for biological fluid, **characterized in that** it comprises:
- a second connector (22) capable of being reversibly attached in a leaktight manner to a first connector (16) of a sampler device (11) according to any one of the preceding claims,
- means (23) which serve for transferring transfer fluid and which open into said second connector (22).

5. Dispensing device according to Claim 4, additionally comprising injection means capable of pushing a predefined volume of transfer fluid through the means (23) for transferring transfer fluid in the direction of the second connector (22).

6. Dispensing device according to Claim 5, in which the injection means comprise a syringe.

7. Dispensing device according to one of Claims 4 to 6, additionally comprising a measuring container (41) arranged in such a way as to be able to directly receive fluid issuing from a capillary component (13) of a sampler device (11) attached to the second connector (22).

8. Dispensing device according to Claim 7, additionally comprising displacement means (40) capable of positioning the second connector (22):
- in a first position, in such a way as to allow a sampler device (11) to be attached to and removed from said second connector (22),
- in a second position, in such a way that a transfer fluid issuing from a sampler device (11) inserted in said second connector (22) is able to flow into the measuring container (41).

9. Dispensing device according to Claim 8, in which the displacement means (40) comprise means of rotation.

10. Dispensing device (21) according to any one of Claims 4 to 9, comprising a measuring container (41) and a first receptacle (42), said dispensing device (21) being portable.

11. Portable dispensing device (21) according to the preceding claim, **characterized in that** the first receptacle (42) comprises injection means that are capable of pushing the liquid contained in said first receptacle (42) exclusively through the fluid conduit (23) and the capillary component (13).

12. Portable dispensing device (21) according to either of Claims 10 and 11, **characterized in that** it additionally comprises an interfacing means (63) capable of connecting said portable dispensing device (21) at least mechanically to another analysis device.

13. Portable dispensing device (21) according to the preceding claim, **characterized in that** the interfacing means (63) additionally comprises a fluid connection.

14. Apparatus for analysis of biological fluids, **characterized in that** it comprises a dispensing device for biological fluid according to one of Claims 4 to 13.

15. Apparatus according to Claim 14, intended to analyze a biological fluid that comprises blood.

16. Method for sampling and dispensing a biological fluid, said method using a sampler device (11) according to either of Claims 2 and 3, provided with a capillary component (13), and a dispensing device (21) according to one of Claims 4 to 13, **characterized in that** it comprises a step of transfer and dilution of a sample of biological fluid contained in said capillary component (13) by injecting a predefined volume of transfer fluid through the means (23) for transferring transfer fluid and through said capillary component (13) .

17. Sampling and dispensing method according to Claim 16, comprising a step of transferring the sample of biological fluid and a defined volume of transfer fluid into a measuring container (41), in such a way as to obtain, in said measuring container (41), a dilution of the biological fluid that is directly adapted to a biological analysis operation.

18. Sampling and dispensing method of either of Claims 16 and 17, comprising a preliminary step of collecting a sample of biological fluid by capillary action in a capillary component (13) of a sampler device (11).

19. Sampling and dispensing method according to one of Claims 16 to 18, implemented with a biological fluid that comprises blood.

20. Sampling and dispensing method according to Claim 19, comprising a step of transferring a defined volume of transfer fluid suitable for obtaining a dilution directly adapted to at least one of the following biological analysis operations:
- counting of cells,
- counting of formed elements,
- dosing of an analyte present in the biological fluid,
- cell characterization.
